(19) Europäisches Patentamt
European Patent Office
Office européen des brevets

(11) Veröffentlichungsnummer : **0 338 430 B1**

# (12) EUROPÄISCHE PATENTSCHRIFT

(45) Veröffentlichungstag der Patentschrift :
**22.07.92 Patentblatt 92/30**

(51) Int. Cl.$^5$ : **C07C 279/12,** A01N 47/44

(21) Anmeldenummer : **89106603.7**

(22) Anmeldetag : **13.04.89**

## (54) Guanidiniumverbindungen und diese enthaltende Fungizide.

(30) Priorität : **19.04.88 DE 3812945**

(43) Veröffentlichungstag der Anmeldung :
**25.10.89 Patentblatt 89/43**

(45) Bekanntmachung des Hinweises auf die Patenterteilung :
**22.07.92 Patentblatt 92/30**

(84) Benannte Vertragsstaaten :
**AT BE CH DE ES FR GB GR IT LI NL SE**

(56) Entgegenhaltungen :
**EP-A- 0 155 509**
**GB-A- 1 294 443**
**US-A- 4 694 086**
**CHEMICAL ABSTRACTS, Band 89, Nr. 19, 6. November 1978, Seite 175, Zusammenfassung Nr. 158758a, Columbus, Ohio, US; &JP-A-78 41 426**

(73) Patentinhaber : **BASF Aktiengesellschaft**
**Carl-Bosch-Strasse 38**
**W-6700 Ludwigshafen (DE)**

(72) Erfinder : **Zipplies, Matthias, Dr.**
**Kastanienweg 1**
**W-6945 Hirschberg (DE)**
Erfinder : **Sauter, Hubert, Dr.**
**Neckarpromenade 20**
**W-6800 Mannheim 1 (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 13**
**W-6730 Neustadt (DE)**

**EP 0 338 430 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Dodecylbenzolsulfonate von guanidiertem Bis-(6-aminohexyl)amin, deren Verwendung als Fungizide, diese enthaltende Fungizide sowie Verfahren zur Bekämpfung von Pilzen mit diesen Verbindungen.

Aus GB-1 114 155 ist die Verbindung

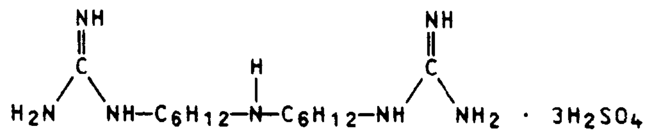

und ihre fungizide Wirkung bekannt.

Die Verbindung zeigt aber in der Praxis nur eine unbefriedigende fungizide Wirkung gegen pflanzenpathogene Pilze. Außerdem ist sie stark phytotoxisch gegenüber Nutzpflanzen.

Aus der EP-155 509 ist die Verbindung

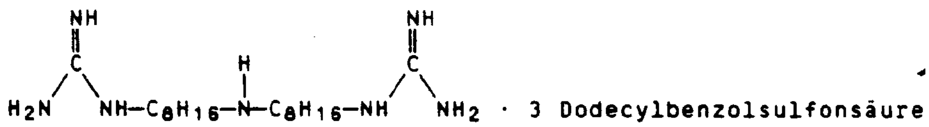

und ihre fungizide Wirkung bekannt.

Die Verbindung besitzt zwar eine etwas besser Pflanzenverträglichkeit, ist aber nur sehr wenig wasserlöslich.

Es besteht daher die Aufgabe, die pflanzenschädigende Wirkung der Verbindungen zu senken, ihre Wasserlöslichkeit zu verbessern und dadurch ihre Verwendungsmöglichkeit als Fungizide in der Landwirtschaft zu verbessern.

Es wurde nun gefunden, daß die Guanidiniumdodecylbenzolsulfonate der Formel I

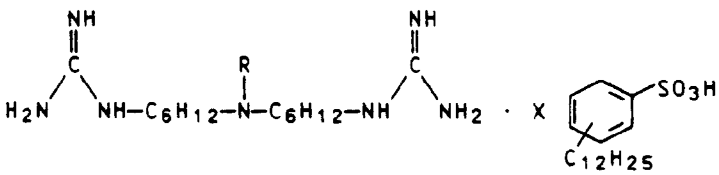

I

in der
R die Gruppe -H,

$-CH_3$ oder 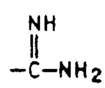

und X die Zahl 1,2,3 oder 4 bedeutet, eine sehr viel bessere Pflanzenverträglichkeit besitzen als die bekannten Verbindungen und eine gute Wirkung gegen phytopathogene Pilze aufweisen. Die verminderte Phytotoxizität der erfindungsgemäßen Verbindungen ermöglicht ihren Einsatz zur Bekämpfung von Schadpilzen in Kulturen von empfindlichen Nutzpflanzen.

Beispiele hierfür sind Schorf (Ventura inaequalis) und Apfelmehltau (Podosphaera leucotricha) bei Äpfeln, Echter Mehltau (Uncinula necator) und Grauschimmel (Botrytis cinerea) bei Reben.

Unter der Dodecylbenzolsulfonsäure ist sowohl eine reine Dodecylbenzolsulfonsäure als auch ein handelsübliches Isomerengemisch von Dodecylbenzolsulfonsäure zu verstehen. Das Isomerengemisch kann auch geringe Mengen homologer Verbindungen enthalten, die an Stelle des Dodecylrestes einen längeren oder kürzeren Alkylrest enthalten.

Beispiele für erfindungsgemäße pflanzenverträgliche fungizide Guanidin-Verbindungen sind das N,N'''-

(Iminodi-6,1-hexanediyl)bis-guanidin-tris-Dodecylbenzol-sulfonat der Formel II
sowie das
N,N'''-Bis[6(aminoiminomethyl)amino]hexyl-guanidin-tris-Dodecyl-benzolsulfonat der Formel III. Die Hexylreste sind n-Hexylreste.

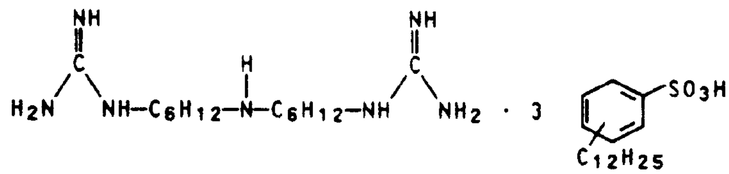

II

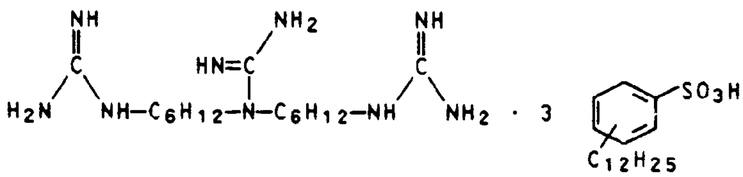

III

Die erfindungsgemäßen Verbindungen lassen sich beispielsweise herstellen, indem man eine Lösung eines Säureadditionssalzes, bestehend aus guanidiertem Bis-(6-aminohexyl)amin und einer anorganischen oder niedermolekularen organischen Säure wie beispielsweise Salzsäure, Schwefelsäure, Phosphorsäure, Kohlensäure, Ameisensäure, Essigsäure, Propionsäure, Oxalsäure mit 1 bis 4 Äquivalenten Dodecylbenzolsulfonsäure in einem Lösungsmittel wie Wasser, Methanol, Ethanol, Isopropanol, Aceton oder auch Mischungen davon bei 20-120°C umsetzt und das Reaktionsprodukt beispielsweise durch Aufkonzentrierung, Kühlung oder Fällung isoliert.

Als Säureadditionssalze bevorzugt werden die Carbonate.

Die erfindungsgemäßen Verbindungen lassen sich beispielsweise außerdem herstellen, indem man eine Lösung eines der oben beschriebenen Säureadditionssalze mit einem Anionenaustauscherharz reagieren läßt und das gebildete freie Guanidin-Derivat mit 1 bis 4 Äquivalenten Dodecylbenzolsulfonsäure in einem Lösungsmittel wie Wasser oder Methanol oder Ethanol umsetzt.

Vorteilhaft lassen sich die Verbindungen außerdem beispielsweise durch Reaktion von Bis(6-aminohexyl)amin mit 2 bis 4 Äquivalenten Cyanamid und 1-4 Äquivalenten Dodecylbenzolsulfonsäure in einem Lösungsmittel wie Wasser, Methanol, Ethanol oder Isopropanol oder Gemischen davon bei 20-90°C und pH 3-10 herstellen.

Das folgende Beispiel erläutert die Herstellung der erfindungsgemäßen Verbindungen.

Beispiel 1

N,N'''-(Iminodi-6,1-hexandiyl)bis-guanidin-tris-Dodecylbenzol-sulfonat (Verbindung II)

100 g N,N'''-(Iminodi-6,1-hexandiyl)bis-guanidin-sulfat (gemäß GB-1,114,155), gelöst in 500 ml Wasser werden bei 70°C mit einer Lösung von 170 g Natriumcarbonat in 340 ml heißem Wasser versetzt. Die Mischung wird mit Eis gekühlt und filtriert. Der Rückstand wird im Vakuum getrocknet. Man erhält 53 g N,N'''-(iminodi-6,1-hexandiyl)bis-guanidinsesquicarbonat. mit dem Fp. 171 - 175°C.

15 g (38 mol) des Sesquicarbonats werden in 100 ml Methanol in der Hitze gelöst, mit 37,5 g Dodecylbenzolsulfonsäure (Isomerengemisch) versetzt und bis zur Beendigung der $CO_2$-Entwicklung gerührt. Das Lösungsmittel wird im Vakuum vollständig abgedampft. Man erhält 52.5 g der Titelverbindung II in Form eines zähen Harzes.

$^1$H-NMR ($CD_3OD$) : δ = 7.8-7.7 (m,6H); 7.3-7.18 (m,6H); 3.17 (t,4H); 2.98 (t,4H); 1.8-0.7 (m,91H).

IR (Film): 3341, 3181, 2956, 2924, 2854, 1671, 1215, 1188, 1125, 1036 $cm^{-1}$.

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse

- wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Fusarium- und Verticillium-Arten an verschiedenen Pflanzen,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. II mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. III werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. II werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. III werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. II werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen.

Durch feines Verteilen der Mischung in Wasser erhält man eine Spritzbrühe.

VI. 3 Gew.-Teile der Verbindung Nr. III werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. II werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. III werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. II werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäureharnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiel

Als bekannte Vergleichswirkstoffe wurden die Verbindungen

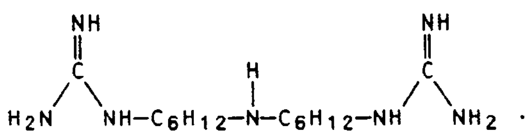 · $3H_2SO_4$

(A) — bekannt aus GB 1 114 155 — und

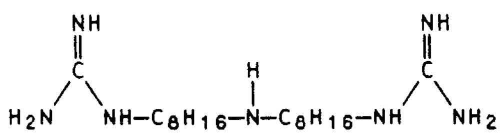 · 3 Dodecylbenzolsulfonsäure

(B) — bekannt aus EP 155 509 —

verwendet.

Wirksamkeit gegen Cercospora arachidicola

Erdnußpflanzen der Sorte "Floorrunner" wurden im 5-6-Fiederblattstadium mit wäßrigen Wirkstoffaufbereitungen, die 80% (Gew.%) Wirkstoff und 20 % Emulgiermittel in der Trockensubstanz enthielten, bis zur Tropfnäße besprüht. Etwa 20 Stunden später wurden die Pflanzen mit wäßriger Sporensuspension von Cercospora arachidicola inokuliert und in ein Gewächshaus mit hoher Luftfeuchtigkeit bei Temperaturen zwischen 23 und 26°C gestellt. Nach zweiwöchiger Kultur konnte das Ausmaß des Befalls ausgewertet werden.

Das Ergebnis zeigt, daß der Wirkstoff gemäß Beispiel 1 bei der Anwendung als 0,05 %ige (Gew.%) Spritzbrühe eine bessere fungizide Wirkung (97 %) zeigt als die bekannten Vergleichswirkstoffe A (80 %) und B (90 %).

## Patentansprüche

1. Guanidinium-Dodecylbenzolsulfonate der Formel I

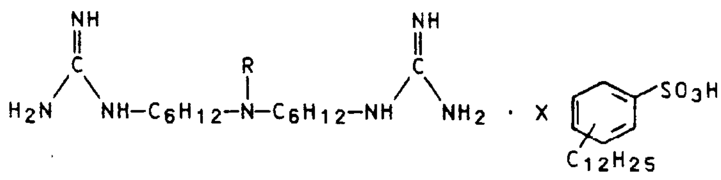 I

in der
R die Gruppe -H,

$-CH_3$ oder 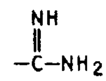

und
X die Zahl 1, 2, 3 oder 4 bedeutet.

2. Fungizid, enthaltend einen festen oder flüssigen Trägerstoff und eine fungizid wirksame Menge eines Guanidinium-Dodecylbenzolsulfonats der Formel I

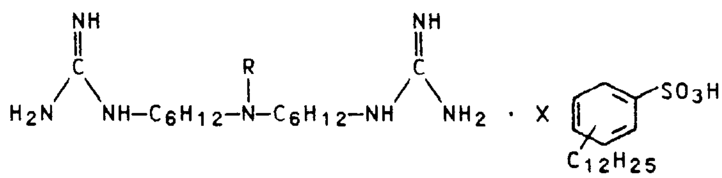 I

in der
R die Gruppe -H,

$-CH_3$ oder 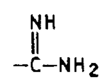

und
X die Zahl 1, 2, 3 oder 4 bedeutet.

3. Verwendung einer Verbindung der Formel I gemäß Anspruch 1 als Fungizid.

4. Verfahren zur Bekämpfung von Pilzen, dadurch gekennzeichnet, daß man die Pilze oder die vom Pilzbefall bedrohten Materialien, Pflanzen, Saatgüter oder den Boden mit einem Guanidinium-Dodecylbenzolsulfonat der Formel I

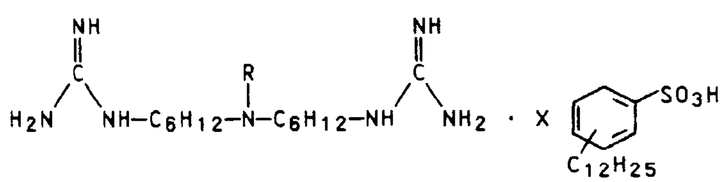 I

in der
R die Gruppe -H,

$-CH_3$ oder 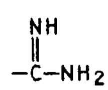

und X die Zahl 1, 2, 3 oder 4 bedeutet, behandelt.

5. Verwendung von Mischungen einer Verbindung der Formel I gemäß Anspruch 1 mit anderen bekannten

fungiziden Verbindungen als Fungizide.

6. N,N'''-(Iminodi-6,1-hexanediyl)bis-guanidin-tris-Dodecylbenzolsufonat.

7. N,N'''-Bis[6(aminoiminomethyl)amino]hexyl-guanidin-tris-Dodecylbenzolsulfonat.

## Revendications

1. Dodecylbenzènesulfonate de guanidinium de la formule I

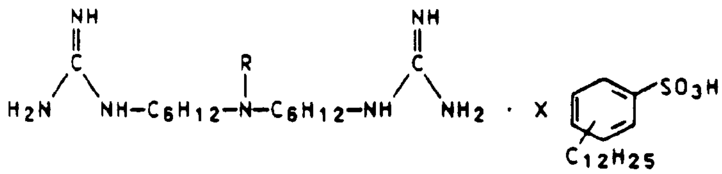

I

dans laquelle
R représente le groupe -H,

$-CH_3$ ou 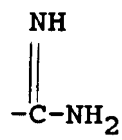

et
X le nombre 1, 2, 3 ou 4.

2. Fongicide, contenant un véhicule solide ou liquide et une quantité à activité fongicide d'un dodecylbenzènesulfonate de guanidinium de la formule I

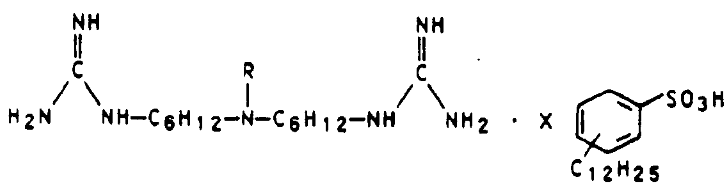

I

dans laquelle
R représente le groupe -H,

$-CH_3$ ou 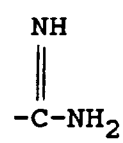

et
X le nombre 1, 2, 3 ou 4.

3. Utilisation d'un composé de la formule I selon la revendication 1 comme fongicide.

4. Procédé de lutte entre les champignons, caractérisé par le fait que l'on traite les champignons ou les matériaux, plantes, semences, menacées par une attaque par des champignons, ou le sol, avec un dodecylbenzènesulfonate de guanidium de la formule I

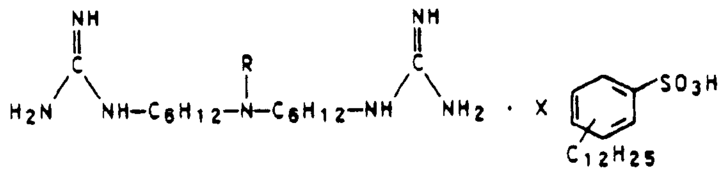

I

dans laquelle
R représente le groupe -H,

$-CH_3$ ou 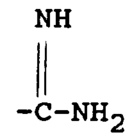

et X le nombre 1, 2, 3 ou 4.

5. Utilisation, comme fongicides, de mélanges d'un composé de la formule I selon la revendication 1 avec d'autres composés fongicides connus.

6. N,N'''-(Iminodi-6,1-hexanediyl) bis-guanidin-tris-dodecylbenzènesulfonate

7. N,N'''-bis[6(aminoiminomethyl)amino]hexyl-guanidin-tris-dodecyl-benzènesulfonate.

## Claims

1. A guanidinium dodecylbenzenesulfonate of the formula I

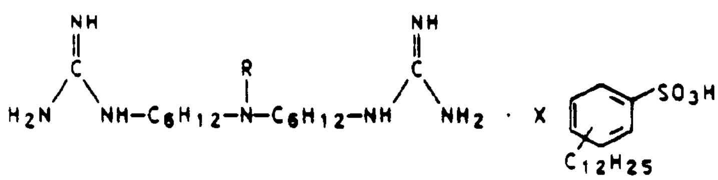

I

where
R is -H, $-CH_3$ or

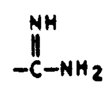

and
X is 1, 2, 3 or 4.

2. A fungicide containing a solid or liquid carrier and a fungicidal amount of a guanidinium dodecylbenzenesulfonate of the formula I

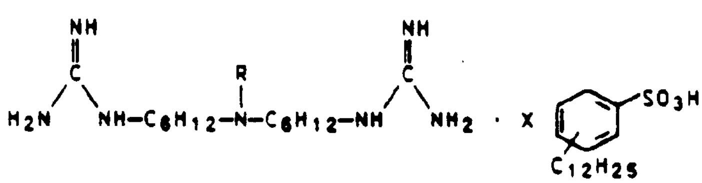

I

where
R is -H, $-CH_2$ or

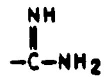

and
X is 1, 2, 3 or 4.

3. Use of a compound of the formula I as claimed in claim 1 as a fungicide.

4. A method for controlling fungi, wherein the fungi or the materials, plants or seeds threatened by fungal attack or the soil is or are treated with a guanidinium dodecylbenzenesulfonate of the formula

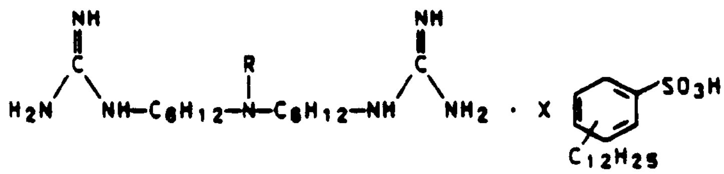

I

where
R is -H, $-CH_3$ or

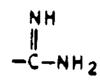

and X is 1, 2, 3 or 4.

5. Use of a mixture of a compound of the formula I as claimed in claim 1 with other known fungicidal compounds as a fungicide.

6. N,N'''-(Iminodi-6,1-hexanediyl)-bisguanidinium trisdodecylbenzenesulfonate.

7. N,N'''-Bis[6-(aminoiminomethyl)-amino]-hexyl-guanidinium trisdodecylbenzenesulfonate.